# EUROPEAN PATENT APPLICATION

(11) **EP 4 582 094 A1**
(43) Date of publication of application: **09.07.2025**
(21) Application number: 23860241.1
(22) Date of filing: 28.08.2023
(51) Int. Cl.: A61K 38/06, A01N 1/02, A61P 9/10, A61P 11/00, A61P 43/00

(54) **ISCHEMIA-REPERFUSION INJURY INHIBITOR**

(30) Priority: 30.08.2022 JP 2022136606
(71) Applicant: Tohoku University, Sendai-shi, Miyagi 980-8577 (JP)
(72) Inventor: OKADA Yoshinori, Sendai-shi, Miyagi 980-8577 (JP); HAYASAKA Kazuki, Sendai-shi, Miyagi 980-8577 (JP); AKAIKE Takaaki, Sendai-shi, Miyagi 980-8577 (JP); MOTOHASHI Hozumi, Sendai-shi, Miyagi 980-8577 (JP); WATANABE Tatsuaki, Sendai-shi, Miyagi 980-8577 (JP); SAKAI Chikara, Sendai-shi, Miyagi 980-8577 (JP)
(74) Representative: Hoffmann Eitle
(86) International application number: PCT/JP2023/030845
(87) International publication number: WO 2024/048479

(57) **Abstract**

The invention relates to inhibition of ischemia-reperfusion injury using a glutathione persulfide and to use in lung transplantation and treatment of pulmonary edema. Specifically, the invention relates to an ischemia-reperfusion injury inhibitor or a pharmaceutical composition for treating or preventing pulmonary edema containing a glutathione persulfide represented by formula (1) below or a pharmaceutically acceptable salt or solvate thereof.

GS(S)ₙG··· (1)

(In the formula, G represents a part of glutathione (GSH) except for a thiol group, and n is an integer of two or more.)

## Description

### Technical Field

### Related Patent

The present application claims priority from a Japanese patent application No. 2022-136606 (filed on August 30, 2022), and the contents thereof are incorporated in the present specification by reference.

### Technical Field

The present invention relates to inhibition of ischemia-reperfusion injury using a glutathione persulfide and to use in lung transplantation and treatment of pulmonary edema.

### Background Art

Lung transplantation is an effective therapeutic method for end stage pulmonary disease, and the number of lung transplantation cases tends to increase every year. The prognosis of lung transplantation, however, is poor compared to that of transplantation of other organs, and the outcomes are not satisfactory.

A major cause of primary graft dysfunction (PGD) and the death after lung transplantation is ischemia-reperfusion injury (IRI). The pathological condition of IRI is pulmonary edema following pulmonary vascular endothelial cell injury, and activation of inflammatory cells such as alveolar macrophages and neutrophils through exposure of the grafted lung to ischemia/reperfusion and subsequent exposure thereof to oxidative stress or inflammatory cytokines contributes to the onset thereof.

It is reported that an antioxidant such as reduced glutathione (GSH), ascorbic acid, SOD and vitamin E is useful for preventing pulmonary edema caused by reperfusion injury after lung transplantation (NPL 1).

Reactive sulfur species containing a sulfur molecule in the compounds are known to play an important role in antioxidative stress response in the living body (NPL 2). Of reactive sulfur species, persulfides having more than one sulfur molecule, such as glutathione trisulfide (GSSSG), have a more potent antioxidative action. It is reported that reactive sulfur specifies have an anti-inflammatory action, inhibit inflammation through TLR4, inhibit activation of NF-κB through IL-1β or IL-6 and inhibit activation of NLRP3 inflammasome.

The inventors have reported that, when a reactive sulfur species is added to an intraocular perfusate, using the antioxidative action and the anti-inflammatory action thereof, the intraocular tissue is protected during ophthalmic surgery and that a highly safe intraocular perfusate can be provided (PTL 1).

### Citation List

### Patent Literature

PTL 1: WO2017/057768

### Non Patent Literature

NPL 1: Katsunobu Kawahara et al., "Reperfusion Injury in Lung and Prevention", Japanese Journal of Vascular Surgery, vol. 1, No. 1, pp. 87-92, 1992

NPL 2: Ida, T. et al. Reactive cysteine persulfides and S-polythiolation regulate oxidative stress and redox signaling. Proc. Natl. Acad. Sci. USA, 2014, doi: 10.1073 / pnas.1321232111

### Summary of Invention

### Technical Problem

A problem of the invention is to provide a novel method for inhibiting ischemia-reperfusion injury.

### Solution to Problem

The inventors have made findings that administration of a glutathione persulfide to a mouse model inhibits ischemia-reperfusion injury (IAI) of a lung and relieves pulmonary edema and the like.

The invention is based on the findings above and relates to [1] to [10] below in a first aspect.
[1] An ischemia-reperfusion injury inhibitor containing a glutathione persulfide represented by formula (1) below or a pharmaceutically acceptable salt or solvate thereof:

   GS(S)nG··· (1)

   (wherein in the formula, G represents a part of glutathione (GSH) except for a thiol group, and n is an integer of two or more.)
   n is preferably an integer of two to five, more preferably an integer of two to four, further preferably two or three.
[2] The inhibitor according to [1], wherein the glutathione persulfide is glutathione trisulfide or glutathione tetrasulfide.
[3] The inhibitor according to [1] or [2] which is used for preventing or treating pulmonary edema.
[4] The inhibitor according to [1] or [2] which is used for assisting organ transplantation.
[5] An organ perfusate or an organ preservation solution containing the inhibitor according to [1] or [2].
[6] The organ perfusate or the organ preservation solution according to [5], wherein the organ is a lung.
[7] A pharmaceutical composition for treating or preventing pulmonary edema containing a glutathione persulfide represented by formula (1) below or a pharmaceutically acceptable salt or solvate thereof:

   GS(S)nG··· (1)

   (wherein in the formula, G represents a part of glutathione (GSH) except for a thiol group, and n is an integer of two or more.)
   n is preferably an integer of two to five, more preferably an integer of two to four, further preferably two or three.
[8] The pharmaceutical composition according to [7], wherein the glutathione persulfide is glutathione trisulfide or glutathione tetrasulfide.
[9] A lung perfusate or a lung preservation solution containing a glutathione persulfide represented by formula (1) below or a pharmaceutically acceptable salt or solvate thereof:

   GS(S)nG··· (1)

   (wherein in the formula, G represents a part of glutathione (GSH) except for a thiol group, and n is an integer of two or more.)
   n is preferably an integer of two to five, more preferably an integer of two to four, further preferably two or three.
[10] The lung perfusate or the lung preservation solution according to [9], wherein the glutathione persulfide is glutathione trisulfide or glutathione tetrasulfide.

The invention relates to [1] to [13] below in a second aspect.
[1] An agent containing a glutathione persulfide represented by formula (1) below or a pharmaceutically acceptable salt or solvate thereof for use in inhibition of ischemia-reperfusion injury:

   GS(S)nG··· (1)

   (wherein in the formula, G represents a part of glutathione (GSH) except for a thiol group, and n is an integer of two or more.)
   n is preferably an integer of two to five, more preferably an integer of two to four, further preferably two or three.
[2] The agent according to [1], wherein the glutathione persulfide is glutathione trisulfide or glutathione tetrasulfide.
[3] The inhibitor according to [1] or [2] which prevents or treats pulmonary edema through inhibition of ischemia-reperfusion injury.
[4] The agent according to [1] or [2] which is used in organ transplantation.
[5] The agent according to [1] or [2] which is used as an organ perfusate or an organ preservation solution or used by adding to an organ perfusate or an organ preservation solution.
[6] The agent according to [4] or [5], wherein the organ is a lung.
[7] A composition for use in treatment or prevention of pulmonary edema which contains a glutathione persulfide represented by formula (1) below or a pharmaceutically acceptable salt or solvate thereof:

   GS(S)nG··· (1)

   (wherein in the formula, G represents a part of glutathione (GSH) except for a thiol group, and n is an integer of two or more.)
   n is preferably an integer of two to five, more preferably an integer of two to four, further preferably two or three.
[8] The composition according to [7], wherein the glutathione persulfide is glutathione trisulfide or glutathione tetrasulfide.
[9] The composition according to [7] or [8] which treats or prevents pulmonary edema through inhibition of ischemia-reperfusion injury.
[10] The composition according to any of [7] to [9], wherein the pulmonary edema is pulmonary edema following ischemia-reperfusion injury, such as pulmonary edema after ischemia-reperfusion injury.
[11] The composition according to any of [7] to [9], wherein the pulmonary edema is pulmonary edema following lung transplantation, such as pulmonary edema after lung transplantation.
[12] The composition according to any of [7] to [11] which is administered to a subject.
[13] The composition according to any of [7] to [12] which is used as a lung perfusate or a lung preservation solution in lung transplantation or used by adding to a lung perfusate or a lung preservation solution in lung transplantation.

The invention relates to [1] to [16] below in a third aspect.
[1] A method for inhibiting ischemia-reperfusion injury in a subject which includes administering a composition containing a glutathione persulfide represented by formula (1) below or a pharmaceutically acceptable salt or solvate thereof to the subject or applying the composition to an organ to be transplanted into the subject:

   GS(S)nG··· (1)

   (wherein in the formula, G represents a part of glutathione (GSH) except for a thiol group, and n is an integer of two or more.)
[2] The method according to [1], wherein n is an integer of two to five.
[3] The method according to [1], wherein the glutathione persulfide is glutathione trisulfide or glutathione tetrasulfide.
[4] The method according to [1], wherein the ischemia-reperfusion injury is ischemia-reperfusion injury following organ transplantation.
[5] The method according to [1], wherein the composition is applied as an organ perfusate or an organ preservation solution for the organ to be transplanted into the subject or applied by adding to an organ perfusate or an organ preservation solution.
[6] The method according to [1], wherein the organ is a lung.
[7] A method for preventing or treating pulmonary edema in a subject
   which includes administering a composition containing a glutathione persulfide represented by formula (1) below or a pharmaceutically acceptable salt or solvate thereof to the subject:

      GS(S)nG··· (1)
   (wherein in the formula, G represents a part of glutathione (GSH) except for a thiol group, and n is an integer of two or more.)
[8] The method according to [7], wherein n is an integer of two to five.
[9] The method according to [7], wherein the glutathione persulfide is glutathione trisulfide or glutathione tetrasulfide.
[10] The method according to [7], wherein the pulmonary edema is pulmonary edema following ischemia-reperfusion injury.
[11] The method according to [7], wherein the pulmonary edema is pulmonary edema following lung transplantation.
[12] A method for preventing pulmonary edema in lung transplantation
   which includes applying a composition containing a glutathione persulfide represented by formula (1) below or a pharmaceutically acceptable salt or solvate thereof to a lung to be transplanted into a subject:

   GS(S)nG··· (1)

   (wherein in the formula, G represents a part of glutathione (GSH) except for a thiol group, and n is an integer of two or more.)
[13] The method according to [12], wherein n is an integer of two to five.
[14] The method according to [12], wherein the glutathione persulfide is glutathione trisulfide or glutathione tetrasulfide.
[15] The method according to [12], wherein the composition is applied as a lung perfusate or a lung preservation solution in lung transplantation or applied by adding to an organ perfusate or an organ preservation solution.
[16] The method according to [12], wherein the pulmonary edema is pulmonary edema following ischemia-reperfusion injury.

### Advantageous Effects of Invention

According to the invention, IRI relief in transplant surgery is relieved, and the prognosis after transplantation is improved. Moreover, use of the organ preservation solution or the organ perfusate of the invention enables application of an organ from a marginal donor and contributes to the solution to the lack of donors.

### Brief Description of Drawings

[FIG. 1] FIG. 1 shows the wet to dry weight ratios which are physiological evaluation of pulmonary edema (FIG. 1a) and the scores of alveolar edema (FIG. 1b) and the edema around the pulmonary arteries (FIG. 1c) which are histopathological evaluation of pulmonary edema.
[FIG. 2] FIG. 2 shows the results of gene expression analysis of inflammatory cytokines (TNFα, IL1b, IL6 and IL10) in lung tissue after IRI.
[FIG. 3] FIG. 3 shows the influence of addition of GSSSG on the mitochondrial membrane potential of HeLa cells.
[FIG. 4] FIG. 4 shows the probabilities of survival after IRI of young mice and old mice (FIG. 4a) and the wet to dry weight ratios which are physiological evaluation of pulmonary edema (FIG. 4b, left: young mice, and right: old mice). The probability of survival of the old mice was lower than that of the young mice, and pulmonary edema became severe/prolonged.
[FIG. 5] FIG. 5 shows the scores of alveolar edema (FIG. 5a) and the edema around the pulmonary arteries (FIG. 5b) which are histopathological evaluation of pulmonary edema. In both figures, the left shows young mice, and the right shows old mice.
[FIG. 6] FIG. 6 shows the results of gene expression analysis by RNA sequencing. The upper part shows genes involved in inflammation, and the lower part shows genes involved in antioxidative stress.
[FIG. 7] FIG. 7 shows the analysis results of gene expression in lung tissue by RT-PCR. In both figures, the left shows young mice, and the right shows old mice (ns: not significant, *: P<0.05, **: P<0.01 (t-test)).
[FIG. 8] FIG. 8 shows the analysis results of expression of proteins involved in the JAK/STAT3 signal transduction pathway in lung tissue by western blotting.
[FIG. 9] FIG. 9 shows the quantitative results of reactive sulfur species after IRI. In all the graphs, the left shows young mice, and the right shows old mice (*: P<0.05 (t-test)).
[FIG. 10] FIG. 10 shows the degrees of ischemia-reperfusion injury in rat lung transplantation. (A) shows the arterial oxygen tension (PaO₂) after blocking the right pulmonary hilum, and (B) shows the weight gain of grafted lung.

### Description of Embodiments

### 1. Glutathione Persulfide

The "glutathione persulfide" is a molecule in which excessive sulfur atoms are attached to glutathione (oxidized type or reduced type), and examples thereof include GSSH, GSSSH, GSSG, GSSSG, GSSSSG and the like.

Glutathione is a tripeptide composed of three amino acids of glutamic acid, cysteine and glycine and is written as GSH. GSSG, in which two glutathione molecules are linked with a disulfide bond, is called oxidized glutathione, and GSH is sometimes called reduced glutathione.

The "glutathione persulfide" used in the invention is an oxidized glutathione persulfide represented by formula (1) below.

GS(S)ₙG··· (1)

(In the formula, G represents a part of glutathione (GSH) except for a thiol group, and n is an integer of two or more.)

In the invention, n is not particularly limited as long as n is an integer of two or more, but n is preferably an integer of two to five, more preferably an integer of two to four, further preferably two or three. The glutathione persulfide represented by formula (1) above is sometimes referred to as the glutathione persulfide of the invention below.

In the invention, preferable glutathione persulfides include glutathione trisulfide (GSSSG), glutathione tetrasulfide (GSSSSG), glutathione pentasulfide (GSSSSSG) and the like. Of these, glutathione trisulfide or glutathione tetrasulfide is preferable, and glutathione trisulfide is particularly preferable.

The glutaione persulfide of the invention may be in the form of a pharmaceutically (pharmacologically and pharmaceutically) acceptable salt (including an ester) or solvate. The pharmaceutically acceptable salt may be a salt with an inorganic acid or an organic acid such as hydrochloric acid, hydrobromic acid, sulfuric acid, nitric acid, phosphoric acid, citric acid, formic acid, fumaric acid, malic acid, acetic acid, succinic acid, tartaric acid, methanesulfonic acid and p-toluenesulfonic acid, a salt with an alkali metal or an alkaline earth metal such as sodium, potassium, calcium and magnesium or a salt with a basic amine or a basic amino acid. The glutaione persulfide may be an ester with an alcohol or a carboxylic acid having one to 10 carbon atoms, preferably methyl alcohol, ethyl alcohol, acetic acid or propionic acid. The pharmaceutically acceptable solvate may be hydrate.

The glutathione persulfide of the invention may be partially reduced and in the form of GS(S)ₘH (m is an integer of one or more) in an environment (for example, in the living body) in the scope in which the purpose of the invention is not impaired.

The glutathione persulfide of the invention can be synthesized according to a known method by reacting a reactive sulfur donor (for example, sodium sulfide, sodium hydrogen sulfide, other sodium sulfides such as disulfide, trisulfide and tetrasulfide, sodium thiosulfate, sulfur element such as S8 or the like) with reduced glutathione. For example, by reacting reduced glutathione and sodium sulfide under argon condition, terminating the reaction with formic acid, then centrifuging, appropriately pre-treating (Sep-Pak column or the like) the obtained supernatant and subjecting the supernatant to HPLC, a glutathione persulfide can be obtained (refer to WO2017/057768 above).

### 2. Ischemia-Reperfusion Injury Inhibitor

In the invention, the glutathione persulfide above is used for inhibiting ischemia-reperfusion injury. "Ischemia-reperfusion injury" is dysfunction of an organ (tissue) caused when the organ falls into ischemia due to surgery (transplantation), cardiac arrest or the like and then the blood flows again. The mechanisms of ischemia-reperfusion injury are deterioration of mitochondrial function due to generation of ROS (reactive oxygen species) after ischemia reperfusion and the consequent decrease in ATP production, which cause cell death and organopathy.

In the invention, the "ischemia-reperfusion injury inhibitor" means a drug having an action of inhibiting or relieving ischemia-reperfusion injury *in vivo* or *ex vivo.* The ischemia-reperfusion injury inhibitor of the invention contains one kind or two or more kinds of the glutathione persulfides of the invention or pharmaceutically acceptable salts or solvates thereof as an active ingredient.

The ischemia-reperfusion injury inhibitor of the invention can be used as a drug which is orally or parenterally administered to a subject to prevent or treat pulmonary edema for example. The pulmonary edema is described in detail in the section "3. Pharmaceutical Composition for Treating or Preventing Pulmonary Edema" below.

The ischemia-reperfusion injury inhibitor of the invention can also be used for assisting organ transplantation. The "organ" is not particularly limited as long as the organ has a risk of ischemia-reperfusion injury in surgery or organ transplantation, and examples thereof include a heart, a liver, a pancreas, a kidney, a small intestine and the like. The organ may be a tissue or a part composing a part of the organ in the scope in which the purpose of the invention is not impaired. The organ as the subject is preferably a lung.

For example, the ischemia-reperfusion injury inhibitor of the invention can be used by adding to an organ preservation solution or an organ perfusate in organ transplantation or as a component of an organ preservation solution or an organ perfusate. The application to an organ preservation solution or an organ perfusate is described in detail in "4. Organ Preservation Solution/Organ Perfusate".

The ischemia-reperfusion injury inhibitor of the invention can also be used for treating or preventing ischemia-reperfusion injury of the subject (recipient) in organ transplantation. For example, the inhibitor is administered during transplantation surgery and/or before the surgery/after the surgery, preferably after ischemia before reperfusion at which the blood flow to the grafted organ is recovered. The dose of the ischemia-reperfusion injury inhibitor of the invention is appropriately set depending on the degree of preservation of the grafted organ or the conditions of the donor and the recipient (for example, the ages and the symptoms), but in general, as the glutathione persulfide, the inhibitor is administered to the subject orally at 0.1 to 1000 mg/kg body weight, preferably at around 0.1 to 100 mg/kg body weight, or parenterally (for example, injection or infusion) at 0.01 to 100 mg/kg body weight, preferably at around 0.1 to 10 mg/kg body weight.

The ischemia-reperfusion injury inhibitor of the invention may contain a pharmaceutically acceptable carrier or additive depending on the purpose of use or the dosage form. Examples of such a carrier or additive include excipients, binders, lubricants, solvents, disintegrating agents, solubilizing agents, suspending agents, emulsifiers, isotonizing agents, stabilizers, antiseptics, antioxidants, corrigents, colorants, buffers, fluidization promoters and the like, but the carrier or the additive is not restricted thereto, and another generally used carrier or additive can be appropriately used.

The excipients include saccharides such as lactose, glucose and D-mannitol, organic excipients such as starch and celluloses including crystalline cellulose, inorganic excipients such as calcium carbonate and kaolin and the like.

The binders include gelatinized starch, gelatin, gum arabic, methyl cellulose, carboxymethyl cellulose, sodium carboxymethyl cellulose, crystalline cellulose, D-mannitol, trehalose, hydroxypropyl cellulose, hydroxypropyl methylcellulose, polyvinylpyrrolidone, polyvinyl alcohol and the like.

The lubricants include stearic acid, fatty acid salts such as a stearate, talc, silicates and the like.

The solvents include purified water, physiological saline, phosphate buffer and the like.

The disintegrating agents include low-substituted hydroxypropyl cellulose, chemically modified cellulose or starch and the like.

The solubilizing agents include polyethylene glycol, propylene glycol, trehalose, benzyl benzoate, ethanol, sodium carbonate, sodium citrate, sodium salicylate, sodium acetate and the like.

The suspending agents or the emulsifiers include sodium lauryl sulfate, gum arabic, gelatin, lecithin, glycerol monostearate, polyvinyl alcohol, polyvinylpyrrolidone, celluloses such as sodium carboxymethyl cellulose, polysorbates, polyoxyethylene hydrogenated castor oil and the like.

The isotonizing agents include sodium chloride, potassium chloride, saccharides, glycerin, urea and the like.

The stabilizers include polyethylene glycol, sodium dextran sulfate, other amino acids, magnesium carbonate, which is also an acidity modifier, and the like.

The antiseptics include paraoxy benzoate esters, chlorobutanol, benzyl alcohol, phenethyl alcohol, dehydroacetic acid, sorbic acid and the like.

The antioxidants include ascorbic acid, which is a water-soluble antioxidant, cysteine hydrochloride, sodium bisulfate, sodium metabisulfite, sodium sulfite, ascorbyl palmitate, which is a fat-soluble antioxidant, butylated hydroxyanisole (BHA), butylated hydroxytoluene (BHT), lecithin, propyl gallate, α-tocopherol, citric acid, which is a metal chelating agent, ethylenediaminetetraacetic acid (EDTA), sorbitol, tartaric acid and phosphoric acid.

The flavoring agents include sweeteners, aromas and the like which are generally used in the field of medicine, and the colorants include colorants which are generally used in the field of medicine.

### 3. Pharmaceutical Composition for Treating or Preventing Pulmonary Edema

The invention provides a pharmaceutical composition for treating or preventing pulmonary edema containing the glutathione persulfide of the invention or a pharmaceutically acceptable salt or solvate thereof.

"Pulmonary edema" is a disease in which liquid components of the blood ooze out of capillary vessels of alveolus, accumulate in the alveolus and thus prevent gas exchange and in which hypoxic condition is thus caused. Causes of pulmonary edema are roughly classified into cardiogenic causes originating in the heart and other noncardiogenic causes. It is known that ischemia-reperfusion injury, especially ischemia-reperfusion injury after lung transplantation, often causes pulmonary edema.

Specific examples to which the pharmaceutical composition of the invention is administered are not limited but include, for example, pulmonary edema following ischemia-reperfusion injury (for example, pulmonary edema after ischemia-reperfusion injury) or pulmonary edema following lung transplantation (for example, pulmonary edema after lung transplantation).

The pharmaceutical composition of the invention inhibits oxidative stress or inflammatory response and also relieves cell dysfunction after ischemia by maintaining mitochondrial function, and thus the pharmaceutical composition can prevent or treat pulmonary edema.

In general, the pharmaceutical composition of the invention is administered to the subject, as the glutathione persulfide, orally at 0.1 to 1000 mg/kg body weight, preferably at around 0.1 to 100 mg/kg body weight or parenterally (for example, injection or infusion) at 0.01 to 100 mg/kg body weight, preferably at around 0.1 to 10 mg/kg body weight. One kind of glutathione persulfide or two or more kinds thereof may be contained.

The pharmaceutical composition of the invention may contain a pharmaceutically acceptable carrier or additive depending on the dosage form or the purpose of use. Examples of such a carrier or additive include excipients, binders, lubricants, solvents, disintegrating agents, solubilizing agents, suspending agents, emulsifiers, isotonizing agents, stabilizers, antiseptics, antioxidants, corrigents, colorants, buffers, fluidization promoters and the like, but the carrier or the additive is not restricted thereto, and another generally used carrier or additive can be appropriately used. The carrier and the additive above are as described in the section above.

### 4. Organ Perfusate/Organ Preservation Solution

The invention provides a pharmaceutical composition for treating or preventing pulmonary edema containing the glutathione persulfide of the invention or a pharmaceutically acceptable salt or solvate thereof.

The organ perfusate is used for perfusing a preserved organ before transplantation, perfusing an organ during transplantation surgery or perfusing a grafted organ after transplantation. The glutathione persulfide concentration contained in the organ perfusate varies with the symptoms of the patient as the subject of use and the kind of the reactive sulfur species but is generally 0.0003 µg/mL or more as the final concentration and may be 0.003 µg/mL or more, 0.01 µg/mL or more, 0.03 µg/mL or more, 0.1 µg/mL or more, 0.3 µg/mL or more, 1 µg/mL or more, 3 µg/mL or more, 10 µg/mL or more or 30 µg/mL or more. The upper limit is generally 1000 µg/mL or less and may be 300 µg/mL or less, 100 µg/mL or less or 30 µg/mL or less. One kind of glutathione persulfide or two or more kinds thereof may be contained.

The pH of the organ perfusate is pH 3 to 10, preferably pH 4 to 9, more preferably pH 6 to 8 or pH 6.5 to 7.5, for example around pH 7.

A component which is generally blended in an organ perfusate may be blended in the organ perfusate in addition to the glutathione persulfide. Such components include electrolytes such as calcium chloride, magnesium chloride, magnesium sulfate, sodium acetate, sodium phosphate, potassium phosphate, sodium citrate and sodium hydrogen carbonate, monosaccharides such as glucose, peptides such as glutathione, antibiotics such as penicillin and the like. The organ preservation solution may contain heparin, an anticoagulant, a protease inhibitor, an immunosuppressive agent, an antioxidant, an anti-inflammatory agent, an antibiotic or the like according to the need in the scope in which the purpose of the invention is not impaired.

The organ perfusate of the invention can be produced according to a known method by dissolving components in sterilized purified water, then adjusting to desired pH using a pH modifier such as hydrochloric acid and sodium hydroxide, filtering with a filter and then sterilizing by heating.

The organ perfusate may also be produced by adding an adequate amount of the glutathione persulfide of the invention to a commercial organ perfusate or organ preservation solution or to a known organ preservation solution such as Collins solution, Euro-Collins solution, University of Wisconsin solution (UW solution), histidine-tryptophan-ketoglutarate (HTK) solution, low potassium dextran (LPD) solution, EP-TU solution, ET-Kyoto solution, St. Thomas solution, Sachs solution, Celsior solution and Belzer solution.

The organ preservation solution of the invention is a solution for preserving a removed organ, and the basic composition thereof is the same as that of the organ perfusate above. The organ preservation solution is used generally at a temperature lower than the body temperature, desirably at -5°C to 25°C, more preferably at 0°C to 20°C, for example in the state of being cooled to 0°C to 10°C.

The "organ" as the subject of the organ perfusate or the organ preservation solution of the invention is not particularly limited as long as the organ has a risk of ischemia-reperfusion injury in surgery or organ transplantation, and examples thereof include a heart, a liver, a pancreas, a kidney, a small intestine and the like. The organ may be a tissue or a part composing a part of the organ in the scope in which the purpose of the invention is not impaired.

The organ is preferably a lung, and a preferable embodiment of the organ perfusate/organ preservation solution of the invention is a lung perfusate/lung preservation solution.

When the organ is preserved or perfused using the organ preservation solution or the organ perfusate of the invention, ischemia-reperfusion injury after transplantation is inhibited or relieved, and graft failure such as pulmonary edema is prevented, resulting in an increase in the success rate of transplantation.

Because ischemia-reperfusion injury following transplantation is caused highly frequently in organ transplantation from an elderly donor, use of the organ preservation solution or the organ perfusate of the invention enables application of an organ from a marginal donor and contributes to the solution to the lack of donors.

### 5. Improvement of Mitochondrial Function

The inventors have observed for the first time that a glutathione persulfide is important for generation of mitochondrial membrane potential and achieves improvement of mitochondrial function and maintenance of mitochondrial function in addition to having an antioxidative or anti-inflammatory action. Known mechanisms of ischemia-reperfusion injury include deterioration of mitochondrial function due to generation of ROS after ischemia reperfusion and the consequent decrease in ATP production. The glutathione persulfide can be considered as a drug which can prevent (inhibit) or treat (relieve) ischemia-reperfusion injury with a different phase from that of conventionally known antioxidants and anti-inflammatory agents.

### Examples

The invention is explained specifically below with Examples, but the invention is not limited to the Examples.

### Example 1: Effect of GSSSG on Ischemia-Reperfusion Injury Materials and Methods

### 1. Preparation of GSSSG Solution

GSSSG (MW 644) was dissolved in 0.1 M HCl to 25 mM GSSSG. Next, 20 µL of 25 mM GSSSG was added to 50 µL of 10x PBS and 430 µL of DW, and thus 500 µL of GSSSG solution (1 mM, 322 µg GSSSG) was adjusted in the end. The pH was found to be between 7.3-7.4.

### 2. Administration of GSSSG

An intervention group (N = 4) in which 500 µL of the GSSSG solution was intraperitoneally administered to C57BL/6J mice (22 months old, male) an hour before pulmonary hilum clamping surgery and a control group (N = 4) in which 500 µL of normal saline solution (NSS) was intraperitoneally administered were set. The pulmonary hilum structure was clamped together for 60 minutes, and the IRI was evaluated an hour and a day after releasing clamping.

### 3. Physiological Evaluation

The wet to dry weight ratios were measured as physiological evaluation of pulmonary edema due to the IRI (N = 4 in each group).

### 4. Histopathological Evaluation

The alveolar edema was scored, and the edema around the pulmonary arteries was quantified as histopathological evaluation of pulmonary edema due to the IRI (N = 6 in each group). The scoring was conducted according to the criteria below, and an average of 10 fields was calculated for each mouse. Regarding the edema around the pulmonary arteries, three to five pulmonary arteries per mouse were extracted, and an average of the area ratios of the pulmonary artery and the edema around the artery was calculated.

### Scoring of Alveolar Edema

0 = no finding
1 = finding in 1-25%
2 = finding in 26-50%
3 = finding in 51-75%
4 = finding in 76-100%

### 5. RT-PCR

The inflammation due to the IRI was evaluated with gene expression of inflammatory cytokines in lung tissue. The wet to dry weight ratios were evaluated with N = 4 in each group, and the other items were evaluated with N = 5-6 in each group.

### Results

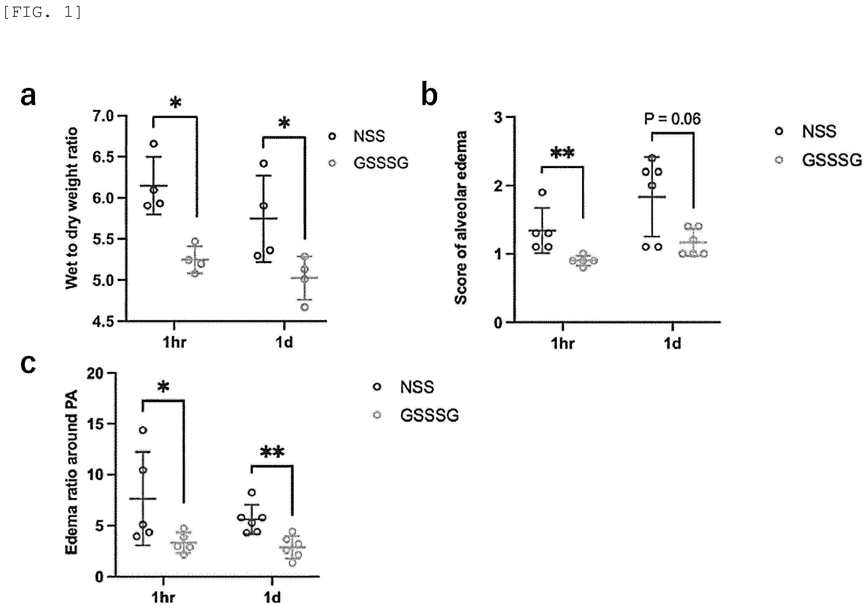

The wet to dry weight ratio, which is a physiological indicator of pulmonary edema, decreased with a significance in the GSSSG administration group compared to that of the control group both an hour and a day after the IRI (FIG. 1a). The scores of the alveolar edema and the edema around the pulmonary arteries, which are histopathological evaluation of pulmonary edema, were examined, and relief of pulmonary edema was observed in the GSSSG administration group in both items (FIGs. 1b and c).

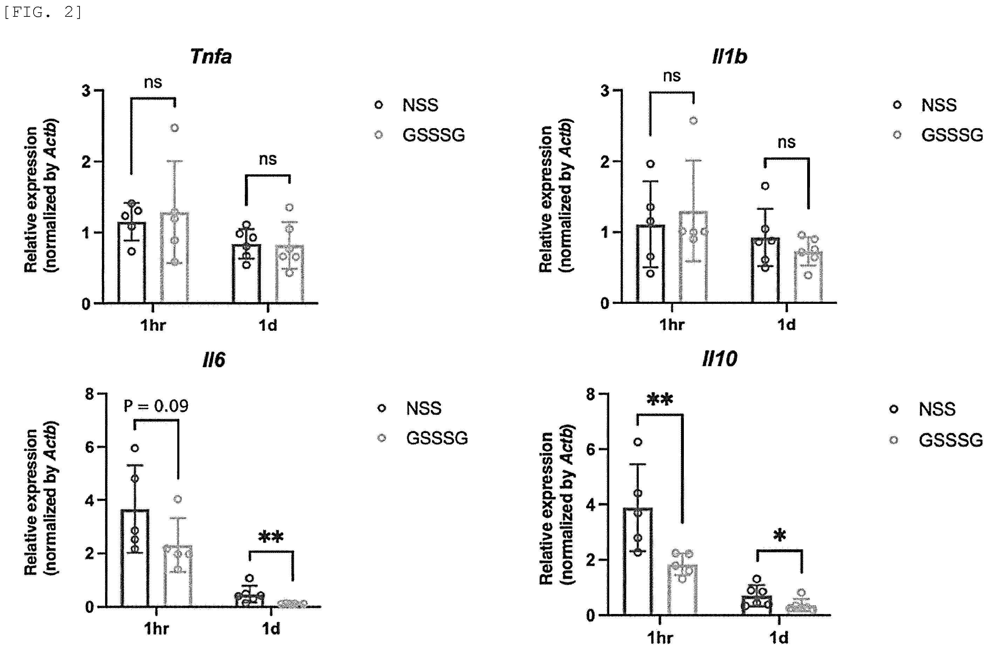

In the evaluation of the inflammatory cytokines in the lung tissue after the IRI, although no difference was observed for TNFα and IL1b, the gene expression of IL6 and IL10 decreased with a significance in the GSSSG administration group (FIG. 2).

### Discussion

It was shown that, through administration of GSSSG, pulmonary edema after lung IRI is relieved, and inflammatory response is inhibited. As the mechanisms, it is believed that a potent antioxidative action and a direct anti-inflammatory action of GSSSG were exhibited. There has been no report so far which demonstrates relief of lung IRI using a persulfide. Although many persulfides are instable as compounds, GSSSG is relatively stable and can be synthesized, and the likelihood that GSSSG is used as a therapeutic agent is high.

### Example 2: Examination of Mitochondrial Membrane Potential Materials and Methods

HeLa cells were cultured in DMEM (low glucose) medium containing 10% FBS, and GSSSG was added to the medium at different concentrations (1 µM, 10 µM and 100 µM). The cells were stained with JC-10, which is a reagent for detecting mitochondrial membrane potential, after 24 hours and observed with a confocal microscope, and the fluorescent intensities were measured with a plate reader.

### Results

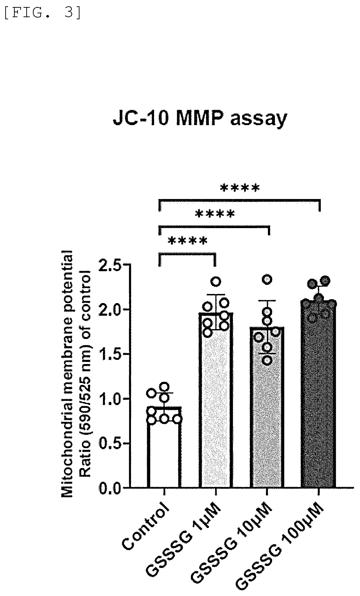

In the examination of the mitochondrial membrane potential using HeLa cells, the mitochondrial membrane potential increased with a significance even with administration of GSSSG at a low concentration (FIG. 3).

### Discussion

It could be observed that GSSSG is important for generation of mitochondrial membrane potential. This suggests that improvement of mitochondrial function is a new mechanism of action of GSSSG. So far, there has been no report regarding mitochondrial function as a mechanism of the cell- and tissue-protecting action of GSSSG. Considering that mechanisms of action of GSSSG are improvement of mitochondrial function of cells, a potent antioxidative action and an anti-inflammatory action, it is expected that administration of GSSSG relieves cell dysfunction after ischemia by maintaining mitochondrial function and relieves oxidative stress and inflammatory response after reperfusion. It is believed that, because GSSSG can approach IRI at different timings in a diversified manner, a high therapeutic effect can be expected.

### Example 3: Comparison of IRI in Old Mice and Young Mice

Because the outcome of lung transplantation from an elderly donor is often poor, it was aimed to elucidate the biological features of IRI specifically found in old mice and the molecular bases thereof using lung IRI models of young mice (two months old) and old mice (22 months old).

Methods and Materials

### 1. Test Design

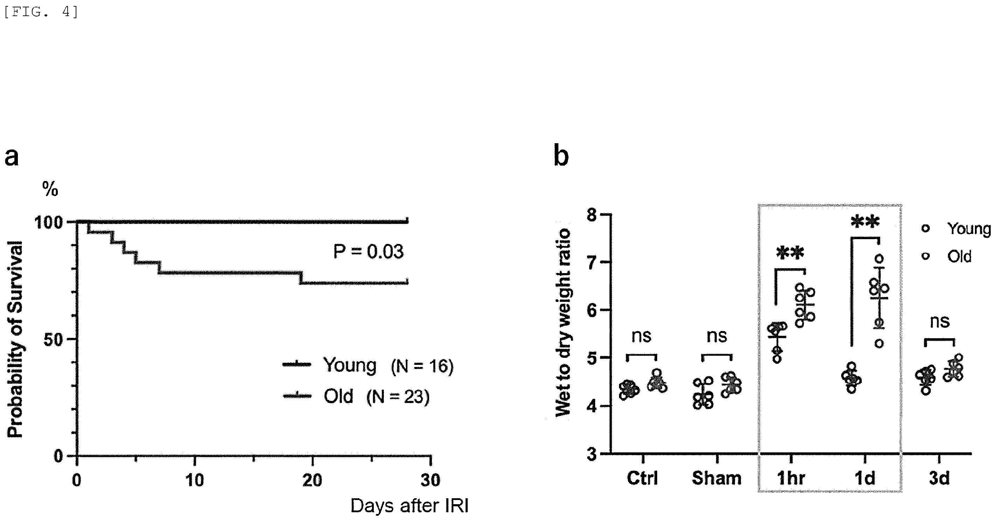

Young (two months old) and old (22 months old) C57BL/6J mice (male) were divided into a control group (N = 6), a Sham group (N = 6) and an IRI group (N = 6). In the IRI group only, the pulmonary hilum structure of the left lung was clamped together for 60 minutes, and the IRI was evaluated an hour, a day, three days and 28 days after releasing clamping. The control group was not treated, and the Sham group was subjected to thoracotomy alone without clamping. The test design is shown in FIG. 4.

### 2. Physiological Evaluation

The wet to dry weight ratios were measured as physiological evaluation of pulmonary edema due to the IRI (N = 6 in each group).

### 3. Histopathological Evaluation

The alveolar edema was scored, and the edema around the pulmonary arteries was quantified as histopathological evaluation of pulmonary edema due to the IRI. The details are as described in Example 1.

### 4. Gene Expression Analysis (RNA Sequencing and RT-PCR)

Using an RNA sequencer (NoVaSeq6000) manufactured by Illumina, Inc., the expression of genes involved in inflammation and antioxidative stress in the left lung following the IRI was analyzed. Moreover, the expression of genes was analyzed by RT-PCR using the total RNA extracted from the left lungs of the IRI group.

### 5. Western Blotting

Western blotting of proteins involved in the JAK/STAT3 signal transduction pathway was conducted.

### 6. Quantification of Reactive Sulfur Species

Reactive sulfur species were detected/quantified using LC-MS/MS according to an existing report (Ida, T. et al. Proc. Natl. Acad. Sci. USA, 2014 above).

### Results

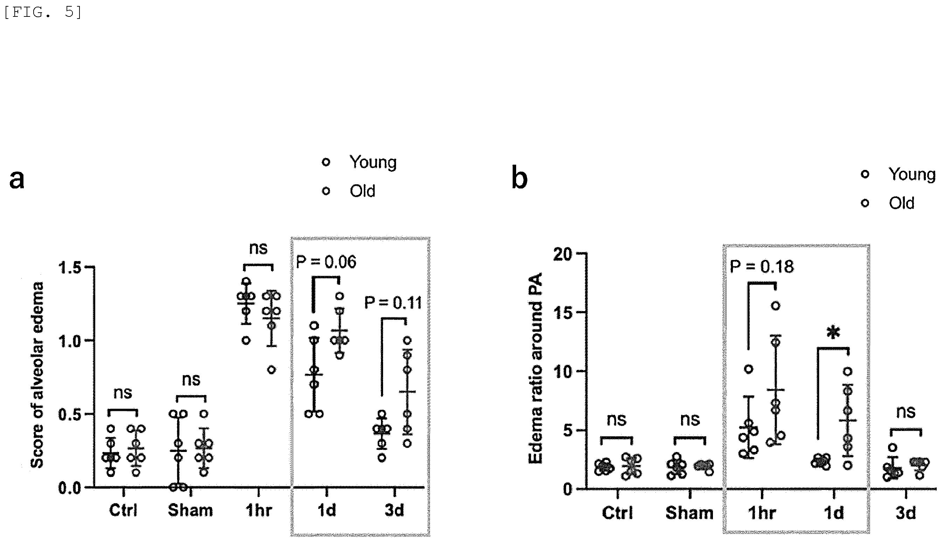

After the IRI, the prognosis of the old mice was poor with a significance compared to that of the young mice, and in particular, many mice died within a week after the IRI (FIG. 4a). Moreover, after the IRI, pulmonary edema became severe/prolonged in the old mice (FIG. 4b). It was also observed that the scores of alveolar edema and the edema around the pulmonary arteries became worse or severe in the old mice compared to those of the young mice (FIGs. 5a and b).

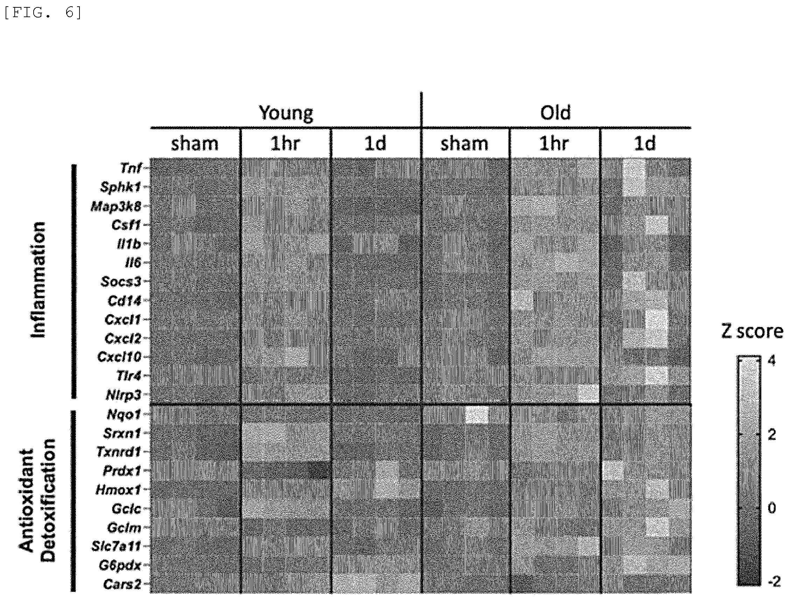

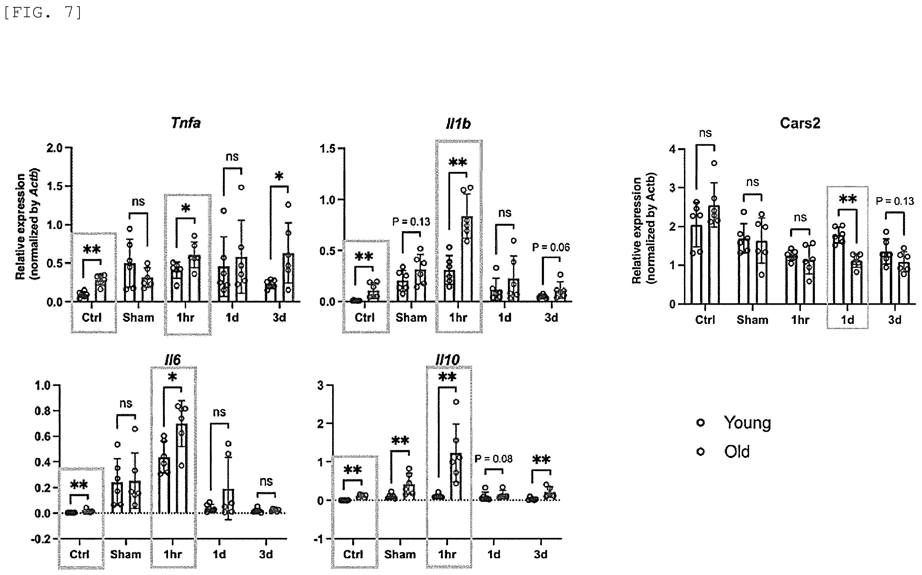

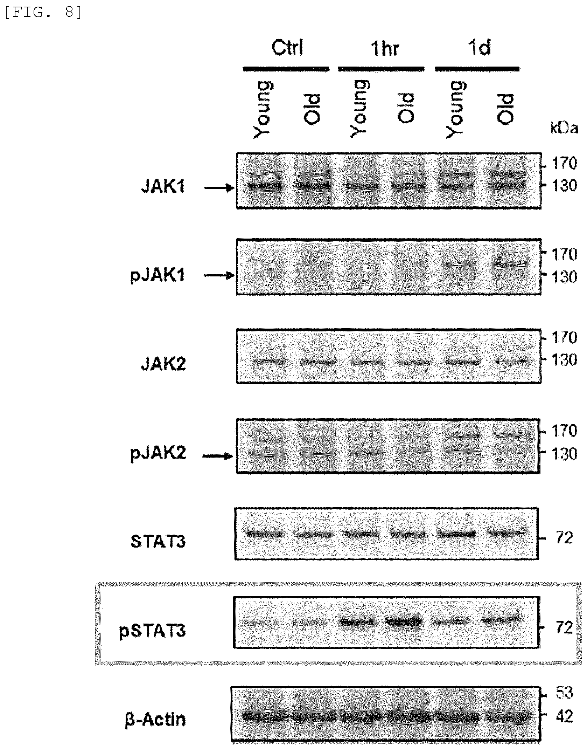

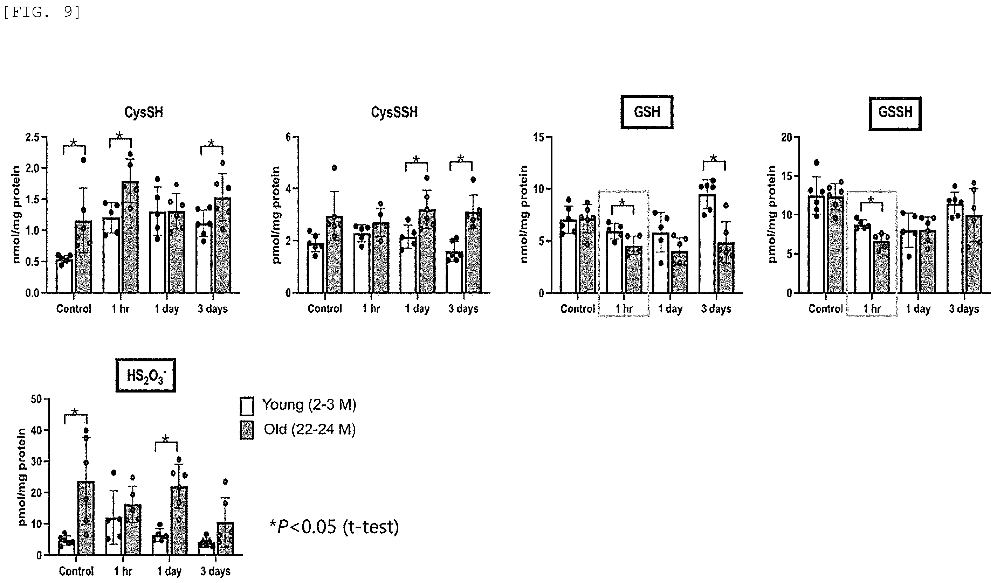

From the RNA sequence analysis, enhancement of the genes involved in inflammation was observed in the old mouse Sham group and the IRI group (1 hr and 1 d) (FIG. 6, top). Moreover, while enhancement of the genes involved in antioxidative stress was observed in the old mouse IRI group (1 d), the expression of Cars2, which is an enzyme that synthesizes a persulfide in mitochondria, decreased (FIG. 6, bottom). From the RT-PCR, in the IRI groups (1 hr), the expression levels of TNFα, IL1b, IL6 and IL10, which are inflammatory cytokines, were higher with a significance in the old mice compared to those of the young mice (FIG. 7). The expression of Cars2 decreased a day after the IRI in the old mice compared to that of the young mice (FIG. 7). Moreover, the expression of pSTAT3, which is involved in the JAK/STAT3 signal transduction pathway, was higher with a significance in the old mice compared to that of the young mice (FIG. 8). Furthermore, a decrease in GSH and GSSH was observed in the old mice after the IRI compared to those of the young mice (FIG. 9).

### Discussion

It is believed that inflammatory response, such as activation of the IL6/JAK/STAT3 pathway or the TNFα pathway through NFκB, is highly involved in the tendency towards deterioration of IRI and in severe IRI in old mice compared to young mice. Here, a decrease in GSH and GSSH was observed in the lungs after the IRI, and the reactive sulfur species are known to inhibit inflammation through TLR4 and inhibit activation of NF-κB through IL-1β and IL-6. Therefore, it is believed that, regarding the improvement of IRI through administration of GSSSG, GSSSG inhibits the activation of the IL6/JAK/STAT3 pathway or NF-κB, instead of decreased GSH and GSSH, and thus relieves IRI.

From the results of the Examples above, it was shown that IRI can be inhibited by adding GSSSG to a perfusate or a preservation solution, and it is expected that IRI in lung transplantation is overcome and that the prognosis after lung transplantation is improved. Administration of GSSSG during preservation of a lung is not limited to during usual lung transplantation but may lead to application of a lung of a marginal donor or relief of IRI during lung transplantation in which ischemic state lasts for a long time. Moreover, in the future, it is expected to be applied to treatment (pre-conditioning) using GSSSG in *ex vivo* lung perfusion (EVLP) in lung transplantation. Administration of GSSSG during lung transplantation may contribute also to the solution to the lack of donors, which is a major problem of lung transplantation.

Ischemia reperfusion is an important issue in preservation of organs not only in lung transplantation but also in organ transplantation in general. Considering the mechanisms of action of GSSSG found by the inventors, an effect on organs other than the lungs (the heart, the livers, the kidneys and the like) can be expected, and the effect of GSSSG of inhibiting IRI may be extended to the entire organ transplantation medicine in the future. Moreover, a similar effect can be also expected from other glutathione persulfides (GS(S)nG, n≥2).

### Example 4: Effect of GSSSG in Rat Isogeneic Lung Transplantation

In rat isogeneic lung transplantation, the degrees of ischemia-reperfusion injury after transplantation were compared between a case in which the lung was preserved in a lung preservation solution containing GSSSG and a case in which the lung was preserved in a lung preservation solution that did not contain GSSSG (control).

### Materials and Methods:

In the GSSSG group (n=3), ET-TU solution containing GSSSG was used as the lung preservation solution. The GSSSG concentration was 300 µM, at which GSSSG could be mixed into EP-TU while maintaining pH 7 in a preliminary experiment. In the control group (n=3), ET-TU solution was used as it was as the lung preservation solution. As the rats, Lewis rats (male, 13-18 weeks, 275-350 g) were used both as the donors and the recipients.

The left lung of a donor was preserved for six hours at 4°C using the lung preservation solution (EP-TU) and transplanted into the left lung of a recipient rat. After reperfusion for an hour after the lung transplantation, the right pulmonary hilum (pulmonary artery and vein/bronchus) was blocked, and the degree of ischemia-reperfusion injury of the left lung was evaluated. As indicators for ischemia-reperfusion injury, the weight gain of the grafted lung (weight after transplantation - weight before transplantation) and the arterial oxygen tension (PaO₂) after blocking the right pulmonary hilum were used.

### Results:

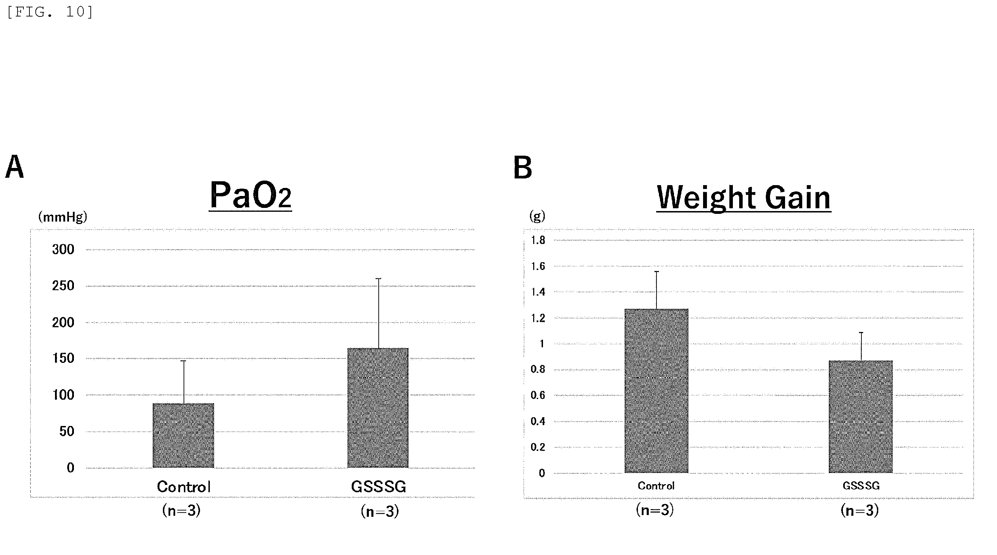

In the GSSSG group, PaO₂ an hour after the reperfusion improved compared to that of the control group (FIG. 10A). Moreover, in the GSSSG group, the weight gain of the grafted lung an hour after the reperfusion decreased compared to that of the control group (FIG. 10B). From the above results, it was observed that addition of GSSSG to a lung preservation solution (EP-TU solution) relieves ischemia-reperfusion lung injury.

### Industrial Applicability

The invention is useful in the field of medical care including organ transplantation, in which ischemia-reperfusion injury causes a problem.

All the publications, the patents and the patent applications cited in the present specification are incorporated as they are in the present specification by reference.

## Claims

1. An ischemia-reperfusion injury inhibitor comprising a glutathione persulfide represented by formula (1) below or a pharmaceutically acceptable salt or solvate thereof:
GS(S)ₙG··· (1)
(wherein in the formula, G represents a part of glutathione (GSH) except for a thiol group, and n is an integer of two or more.)

2. The inhibitor according to claim 1, wherein the glutathione persulfide is glutathione trisulfide or glutathione tetrasulfide.

3. The inhibitor according to claim 1 or 2 which is used for preventing or treating pulmonary edema.

4. The inhibitor according to claim 1 or 2 which is used for assisting organ transplantation.

5. An organ perfusate or an organ preservation solution comprising the inhibitor according to claim 1 or 2.

6. The organ perfusate or the organ preservation solution according to claim 5, wherein the organ is a lung.

7. A pharmaceutical composition for treating or preventing pulmonary edema comprising a glutathione persulfide represented by formula (1) below or a pharmaceutically acceptable salt or solvate thereof:
GS(S)ₙG··· (1)
(wherein in the formula, G represents a part of glutathione (GSH) except for a thiol group, and n is an integer of two or more.)

8. The pharmaceutical composition according to claim 7, wherein the glutathione persulfide is glutathione trisulfide or glutathione tetrasulfide.

9. A lung perfusate or a lung preservation solution comprising a glutathione persulfide represented by formula (1) below or a pharmaceutically acceptable salt or solvate thereof:
GS(S)ₙG··· (1)
(wherein in the formula, G represents a part of glutathione (GSH) except for a thiol group, and n is an integer of two or more.)

10. The lung perfusate or the lung preservation solution according to claim 9, wherein the glutathione persulfide is glutathione trisulfide or glutathione tetrasulfide.
